# EUROPEAN PATENT APPLICATION

(11) **EP 4 286 358 A1**
(43) Date of publication of application: **06.12.2023**
(21) Application number: 22911196.8
(22) Date of filing: 19.12.2022
(51) Int. Cl.: C07C 29/152, B01D 53/22, B01D 61/58, C07B 61/00, C07C 1/12, C07C 31/04, C10L 1/00

(54) **REACTOR AND LIQUID FUEL SYNTHESIS METHOD**

(30) Priority: 22.12.2021 JP 2021208346; 25.08.2022 JP 2022134437
(71) Applicant: NGK Insulators, Ltd., Nagoya-shi, Aichi 467-8530 (JP)
(72) Inventor: NAKAGAWA, Kosuke, Nagoya-shi, Aichi 467-8530 (JP); IIDA, Kazuki, Nagoya-shi, Aichi 467-8530 (JP); KAN, Hirofumi, Nagoya-shi, Aichi 467-8530 (JP); TORII, Atsushi, Nagoya-shi, Aichi 467-8530 (JP)
(74) Representative: Müller-Boré & Partner Patentanwälte PartG mbB
(86) International application number: PCT/JP2022/046746
(87) International publication number: WO 2023/120494

(57) **Abstract**

A reactor (1) includes a separation membrane (30), a first flow path (11), and a second flow path (12). The first flow path (11) is provided on a non-permeation side of the separation membrane (30), and the second flow path (12) is provided on a permeation side of the separation membrane (30). The raw material gas flows through the first flow path (11), and a sweep gas flow through the second flow path (12). In a side view of the separation membrane (30), a direction in which the sweep gas flows through the second flow path (12) is opposite to the direction in which the raw material gas flows through the first flow path (11).

## Description

### TECHNICAL FIELD

The present invention relates to a reactor and a liquid fuel synthesis method.

### BACKGROUND ART

In recent years, reactors have been developed capable of improving, in a conversion reaction of a raw material gas containing hydrogen and carbon dioxide to a liquid fuel such as methanol and ethanol (specifically, a fuel in a liquid state in normal temperature and pressure), a conversion efficiency by separating water vapor generated along with the liquid fuel.

For example, Patent Literature 1 discloses a reactor including a separation membrane, a first flow path provided on a non-permeation side of the separation membrane, and a second flow path provided on a permeation side of the separation membrane. A raw material gas is supplied to the first flow path. A sweep gas for sweeping water vapor that has permeated through the separation membrane is supplied to the second flow path. Due to the sweep gas flowing through the second flow path, reaction heat can be absorbed while water vapor is taken in, and thus the conversion efficiency can be improved due to an equilibration shift effect.

### CITATION LIST

### Patent Literature

Patent Literature 1: JP 2018-008940A

### SUMMARY

### TECHNICAL PROBLEM

Here, in the reactor disclosed in Patent Literature 1, since the direction in which the sweep gas flows through the second flow path is the same as the direction in which the raw material gas flows through the first flow path, the amount of water vapor contained in the sweep gas is increased in the downstream area of the second flow path.

For this reason, water vapor cannot be smoothly moved from the downstream area of the first flow path to the downstream area of the second flow path, and thus water vapor is likely to be mixed with the liquid fuel.

This invention has been made in view of the above-described circumstances, and aims to provide a reactor and a reactor device capable of suppressing water vapor from being mixed with a liquid fuel.

### SOLUTION TO PROBLEM

A reactor according to the present invention includes a separation membrane, a first flow path, a second flow path, and a catalyst. The separation membrane is permeable to a product of conversion reaction of a raw material gas containing at least hydrogen and carbon dioxide to a liquid fuel. The first flow path is provided on a non-permeation side of the separation membrane. The second flow path is provided on a permeation side of the separation membrane. The raw material gas flows through the first flow path. A sweep gas for sweeping water vapor that has permeated through the separation membrane flows through the second flow path. The catalyst is disposed in the first flow path and configured to promote the conversion reaction of the raw material gas to the liquid fuel. In a side view of the separation membrane, a direction in which the sweep gas flows through the second flow path is opposite to the direction in which the raw material gas flows through the first flow path.

### ADVANTAGEOUS EFFECTS

According to the present invention, a reactor and a liquid fuel synthesis method capable of suppressing water vapor from being mixed with a liquid fuel can be provided.

### BRIEF DESCRIPTION OF DRAWINGS

FIG. 1 is a perspective view of a reactor 1 according to an embodiment.
FIG. 2 is a cross-sectional view taken along line A-A in FIG. 1.
FIG. 3 is a cross-sectional view taken along line B-B in FIG. 1.
FIG. 4 is a cross-sectional view taken along line C-C in FIG. 2.
FIG. 5 is a perspective view of a reactor 1a according to the embodiment.
FIG. 6 is a cross-sectional view of the reactor 1a.
FIG. 7 is a schematic view of a reactor device according to the embodiment.
FIG. 8 is a schematic view of the reactor device according to the embodiment.
FIG. 9 is a schematic view showing a configuration of a reactor according to modification 1.
FIG. 10 is a schematic view showing a configuration of the reactor according to modification 1.
FIG. 11 is a cross-sectional view of a first housing according to the embodiment.
FIG. 12 is a cross-sectional view of the first housing according to the embodiment.

### DESCRIPTION OF EMBODIMENTS

Next, embodiments of the present invention will be described with reference to the drawings. However, the drawings are schematic, and ratio or the like of dimensions may differ from an actual one.

### Reactor 1

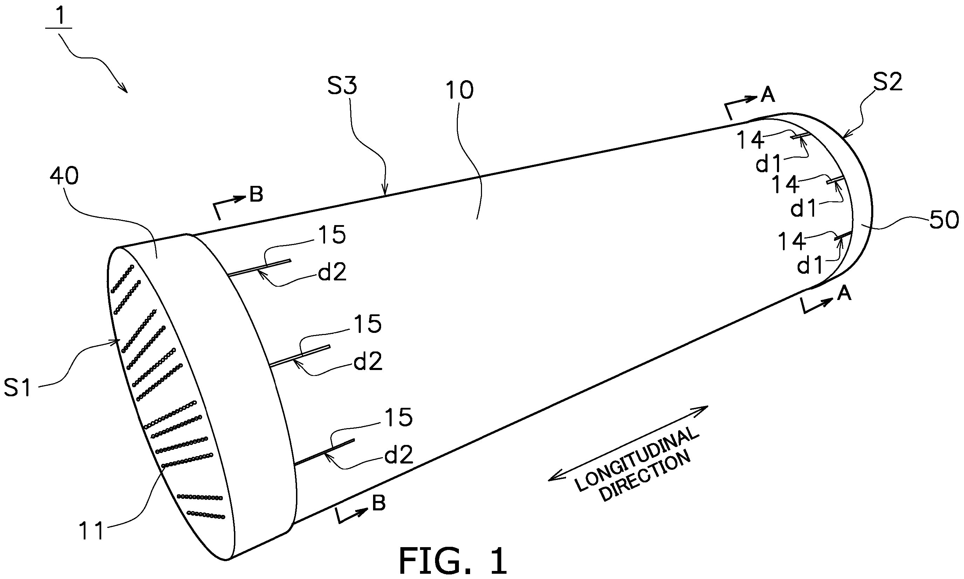

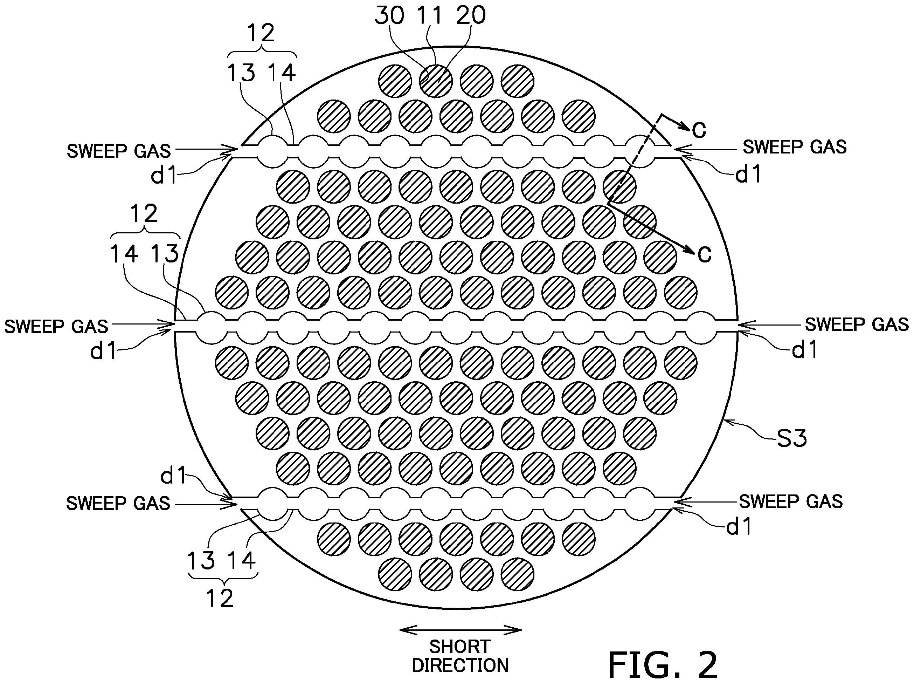

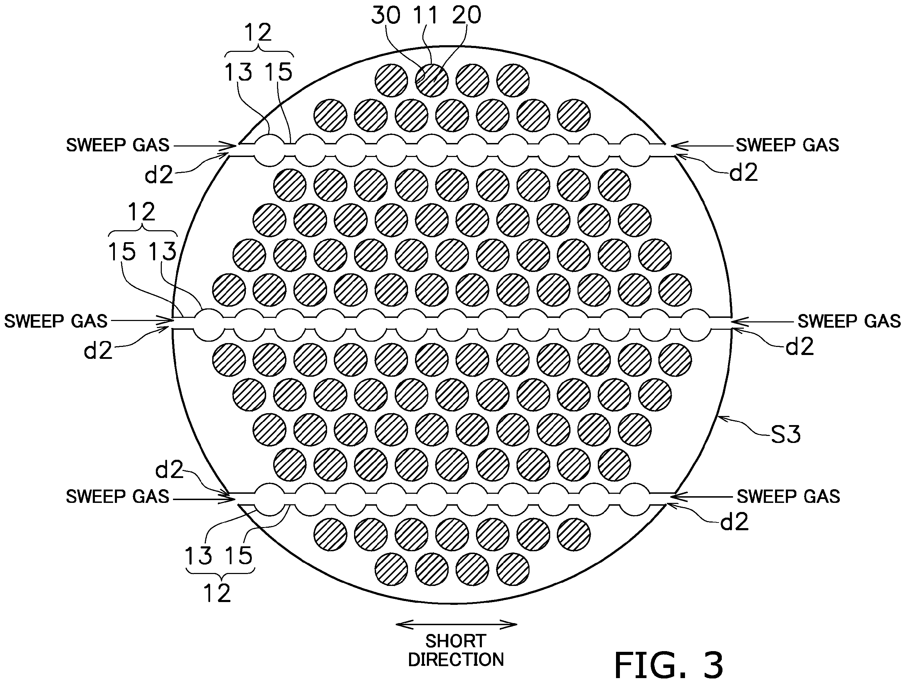

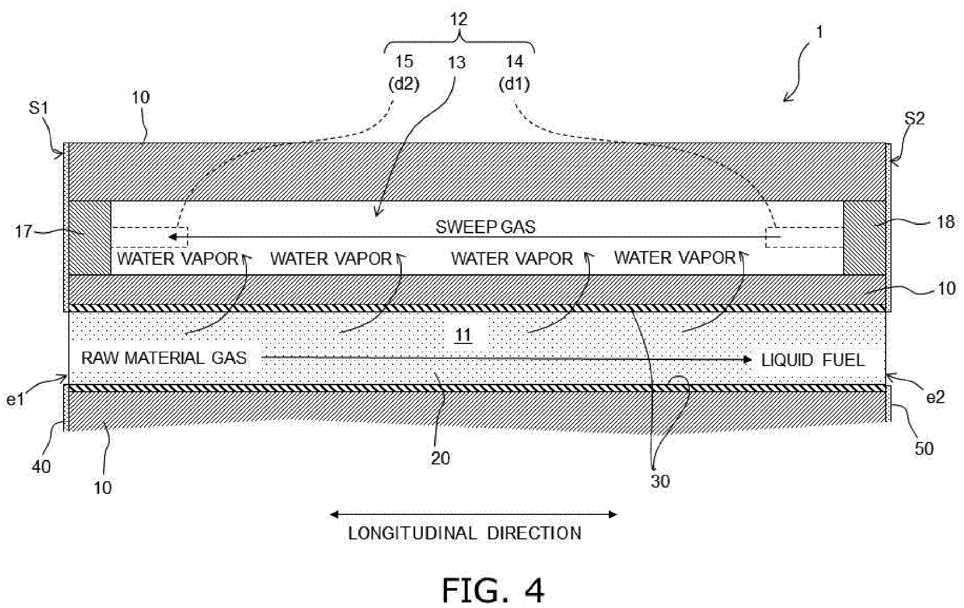

FIG. 1 is a perspective view of a reactor 1. FIG. 2 is a cross sectional view taken along line A-A in FIG. 1. FIG. 3 is a cross sectional view taken along line B-B in FIG. 1. FIG. 4 is a cross sectional view taken along line C-C in FIG. 2.

The reactor 1 is a so-called membrane reactor used to convert a raw material gas to a liquid fuel. The raw material gas contains at least hydrogen and carbon dioxide. The raw material gas may contain carbon monoxide. The raw material gas may be a so-called synthetic gas (syngas). The liquid fuel is a fuel that is in a liquid state at normal temperature and pressure, or a fuel that can be liquidized at normal temperature and pressurized state. Examples of the liquid fuel that is in a liquid state at normal temperature and pressure may include methanol, ethanol, liquid fuels represented by CₙH₂₍ₘ₋₂ₙ₎ (m is an integer less than 90, and n is an integer less than 30), and mixtures thereof. Examples of the fuel that can be liquidized at normal temperature and pressurized state include, for example, propane, butane, and mixtures thereof.

For example, reaction formula (1) for synthesizing methanol by catalytically hydrogenating a raw material gas containing carbon dioxide and hydrogen in the presence of a catalyst is as follows.

CO₂ + 3H₂ ↔ CH₃OH + H₂O (1)

The above reaction is an equilibrium reaction, and in order to increase both the conversion efficiency and the reaction rate, it is preferable to carry out the reactions at a high temperature and high pressure (for example, 180°C or higher and 2 MPa or higher). The liquid fuel is in a gaseous state when it is synthesized, and is kept in a gaseous state at least until it flows out of the reactor 1. The reactor 1 preferably has heat resistance and pressure resistance suitable for desired synthesis conditions of the liquid fuel.

As shown in FIG. 1, the reactor 1 is formed in a monolith shape. A monolith shape means a shape including a plurality of holes that pass through in a longitudinal direction, and is a concept including a honeycomb shape. The reactor 1 includes a first end face S1, a second end face S2, and a side face S3. The first end face S1 is provided on the opposite side to the second end face S2. The side face S3 is continuous with outer edges of the first end face S1 and the second end face S2. Although the reactor 1 is formed in a columnar shape in the present embodiment, the outer shape of the reactor 1 is not particularly limited.

As shown in FIGS. 1 to 4, the reactor 1 includes a porous support body 10, catalysts 20, separation membranes 30, a first seal portion 40, and a second seal portion 50.

The porous support body 10 is a columnar body extending in the longitudinal direction of the reactor 1. The porous support body 10 is constituted by a porous material.

As the porous material, a ceramic material, a metal material, a resin material, or the like can be used, and a ceramic material is particularly preferable. As an aggregate of the ceramic material, for example, at least one of alumina (Al₂O₃), titania (TiO₂), mullite (Al₂O₃-SiO₂), potsherd, cordierite (Mg₂Al₄Si₅O₁₈) can be used. As an inorganic binder for the ceramic material, for example, at least one of titania, mullite, readily sinterable alumina, silica, glass frit, a clay mineral, and readily sinterable cordierite can be used. However, the ceramic material does not need to contain an inorganic binder.

As shown in FIGS. 2 and 3, the porous support body 10 includes a number of first flow paths 11 and a plurality of second flow paths 12.

As shown in FIG. 4, the first flow paths 11 are formed in the longitudinal direction of the reactor 1. The first flow paths 11 are through holes. The first flow paths 11 are open in the first end face S1 and the second end face S2 of the reactor 1. The first flow paths 11 each include an inflow port e1 formed in the first end face S1 and an outflow port e2 formed in the second end face S2. The first flow paths 11 are provided on the non-permeation side of the separation membranes 30. The raw material gas flows through the first flow paths 11. The catalysts 20 are respectively disposed in the first flow paths 11. The number, position, shape, and the like of the first flow paths 11 can be changed as appropriate.

The second flow paths 12 are provided on the permeation side of the separation membranes 30. The sweep gas for sweeping the water vapor that has permeated through the separation membranes 30 flows through the second flow paths 12. An inert gas (e.g., nitrogen), the air, or the like can be used as the sweep gas. The number, position, shape, and the like of the second flow paths 12 can be changed as appropriate.

Here, as shown in FIGS. 2 and 3, the second flow paths 12 are each formed by a plurality of cells 13, an inflow slit 14, and an outflow slit 15.

The plurality of cells 13 are arranged in a row along a short direction (a direction orthogonal to the longitudinal direction) of the reactor 1. As shown in FIG. 4, the cells 13 are formed along the longitudinal direction of the reactor 1. Both ends of each cell 13 are respectively sealed by first and second opening seal portions 17 and 18. The first and second opening seal portions 17 and 18 can be constituted by the above porous material.

As shown in FIG. 1, the inflow slits 14 are formed at one end portion in the longitudinal direction of the reactor 1. The one end portion of the reactor 1 means, when the reactor 1 is equally divided into five in the longitudinal direction, a portion extending to two-fifths from one end on the side from which the liquid fuel flows out. The inflow slits 14 are formed along the short direction of the reactor 1. As shown in FIG. 2, the inflow slits 14 pass through the plurality of cells 13. Both ends of the inflow slits 14 are open in the side face S3. The inflow slits 14 each include a pair of inflow ports d1 formed in the side face S3. The pair of inflow ports d1 correspond to one end of the second flow paths 12 in the longitudinal direction.

As shown in FIG. 1, outflow slits 15 are formed in the other end portion of the reactor 1 in the longitudinal direction. The other end portion of the reactor 1 means, when the reactor 1 is equally divided into five in the longitudinal direction, a portion extending to two-fifths from the other end on the side into which the raw material gas flows in. The outflow slits 15 are formed along the short direction of the reactor 1. As shown in FIG. 3, the outflow slits 15 pass through the plurality of cells 13. Both ends of the outflow slits 15 are open in the side face S3. The outflow slits 15 each include a pair of exhaust ports d2 formed in the side face S3. The pair of exhaust ports d2 correspond to the other ends of the second flow paths 12 in the longitudinal direction.

The catalysts 20 are respectively disposed in the first flow paths 11. Although the catalysts 20 preferably fill the respective first flow paths 11, the catalysts 20 may each be disposed in a layered manner on the surface of each separation membrane 30. As represented by the above formula (1), the catalysts 20 promote the conversion reaction of the raw material gas to the liquid fuel.

As the catalysts 20, a known catalyst suitable for the conversion reaction to a desired liquid fuel can be used. Examples of the catalysts 20 may include metal catalysts (copper, palladium, and the like), oxide catalysts (zinc oxide, zirconia, gallium oxide, and the like), and composite catalysts thereof (copper-zinc oxide, copper-zinc oxide-alumina, copper-zinc oxide-chromium oxide-alumina, copper-cobalt-titania, catalysts obtained by modifying these with palladium, and the like).

The separation membranes 30 are supported by the porous support body 10. The separation membranes 30 surround the respective first flow paths 11. The separation membranes 30 are disposed between the first flow paths 11 and the second flow paths 12.

The separation membranes 30 are permeable to water vapor that is one of products of the conversion reaction of the raw material gas to a liquid fuel. In this manner, by utilizing the equilibrium shift effect, the reaction equilibrium of the above formula (1) can be shifted to the product side.

The separation membranes 30 preferably have a water vapor permeability coefficient of 100 nmol/(s·Pa·m²) or more. The water vapor permeability coefficient can be determined using a known method (see Ind. Eng. Chem. Res., 40, 163-175 (2001)).

The separation membranes 30 preferably have a separation factor of 100 or more. The greater the separation factor is, the more permeable the separation membranes 30 are to water vapor, and less permeable to components other than water vapor (e.g., hydrogen, carbon dioxide, and liquid fuel). The separation factor can be determined using a known method (see FIG. 1 of "Separation and Purification Technology 239 (2020) 116533").

An inorganic membrane can be used as the separation membranes 30. The inorganic membrane is preferable because it has heat resistance, pressure resistance, and water vapor resistance. Examples of the inorganic membrane include a zeolite membrane, a silica membrane, an alumina membrane, and a composite membrane thereof. In particular, an LTA zeolite membrane having a molar ratio (Si/Al) of a silicon element (Si) and an aluminum element (Al) of 1.0 or more and 3.0 or less is suitable because of its excellent water vapor permeability.

As shown in FIG. 1, a first seal portion 40 covers one end face of the porous support body 10. The first seal portion 40 suppresses entry of the raw material gas into the porous support body 10. As shown in FIG. 4, the first seal portion 40 is formed so as not to close the inflow ports e1 of the first flow paths 11. The first seal portion 40 covers the first opening seal portion 17. The first seal portion 40 can be constituted by glass, metal, rubber, resin, or the like.

As shown in FIG. 1, a second seal portion 50 covers the other end face of the porous support body 10. The second seal portion 50 suppresses entry of the liquid fuel into the porous support body 10. As shown in FIG. 4, the second seal portion 50 is formed so as to not close the outflow ports e2 of the first flow paths 11. The second seal portion 50 covers the second opening seal portion 18. The second seal portion 50 can be constituted by glass, metal, rubber, resin, or the like.

### Liquid fuel synthesis method using reactor 1

A liquid fuel synthesis method using the reactor 1 will be described with reference to FIG. 4.

The liquid fuel synthesis method using the reactor 1 includes a step of causing the sweep gas to flow through the second flow paths 12 provided on the permeation side of the separation membranes 30, while causing the raw material gas to flow through the first flow paths 11 provided on the non-permeation side of the separation membranes 30.

The raw material gas flows into the first flow paths 11 through the inflow ports e1 of the first flow paths 11. In the first flow paths 11, water vapor is generated together with the liquid fuel in accordance with the above formula (1). The synthesized liquid fuel flows out through the outflow ports e2 of the first flow paths 11. A residual raw material gas that was not used for the conversion reaction may be mixed with the synthesized liquid fuel. The water vapor, which is a product, permeates through the separation membranes 30 and the porous support body 10, and then moves to the second flow paths 12.

After flowing in through the inflow ports d1 of the inflow slits 14, the sweep gas flows into the cells 13 through the inflow slits 14. Next, the sweep gas that has flowed into the cells 13 through the inflow slits 14 takes in water vapor that has permeated through the separation membranes 30, and flows through the cells 13 toward the outflow slits 15 while absorbing the reaction heat generated by the conversion reaction. The sweep gas that has reached the outflow slits 15 is discharged from the exhaust ports d2 of the outflow slits 15.

Here, as shown in FIG. 4, in the side view of the separation membranes 30, the direction in which the sweep gas flows through the second flow paths 12 is opposite to the direction in which the raw material gas flows through the first flow paths 11. That is, the sweep gas flowing through the second flow paths 12 flows in the opposite direction to the raw material gas flowing through the first flow paths 11.

For this reason, since the amount of water vapor contained in the sweep gas flowing through the upstream area of the second flow paths 12 (specifically, an area on the inflow ports d1 side with respect to the center of the cells 13 in the longitudinal direction) is small, the water vapor can be smoothly moved from the downstream area (specifically, an area on the outflow ports e2 side with respect to the center of the first flow paths 11 in the longitudinal direction) to the upstream area of the second flow paths.

As a result of this, water vapor can be efficiently removed from the liquid fuel flowing out from the first flow path 11, and thus water vapor can be suppressed from being mixed with the liquid fuel, and a liquid fuel of high quality can be obtained.

Note that the direction in which the raw material gas flows through the first flow paths 11 means, when a side of the first flow paths 11 that is close to a raw material gas source is upstream and a side far from the raw material gas source is downstream, the direction that extends from upstream to downstream. Also, the direction in which the sweep gas flows through the second flow paths 12 means, when a side of the second flow paths 12 that is close to a sweep gas source is upstream and a side far from the sweep gas source is downstream, the direction that extends from upstream to downstream.

### Reactor device using reactor 1

The reactor 1 described above can be used in a stand-alone manner, but the reactor 1 can also be coupled with a later-described reactor 1a to form reactor devices 100 and 200. Hereinafter, the reactor 1a, the reactor device 100, and the reactor device 200 will be described in the stated order.

### Reactor 1a

The configuration of the reactor 1a is the same as the configuration of the reactor 1a described above. The difference between the reactor 1a and the reactor 1 is that the sweep gas and the raw material gas flow in the same direction. Hereinafter, the difference will be mainly described.

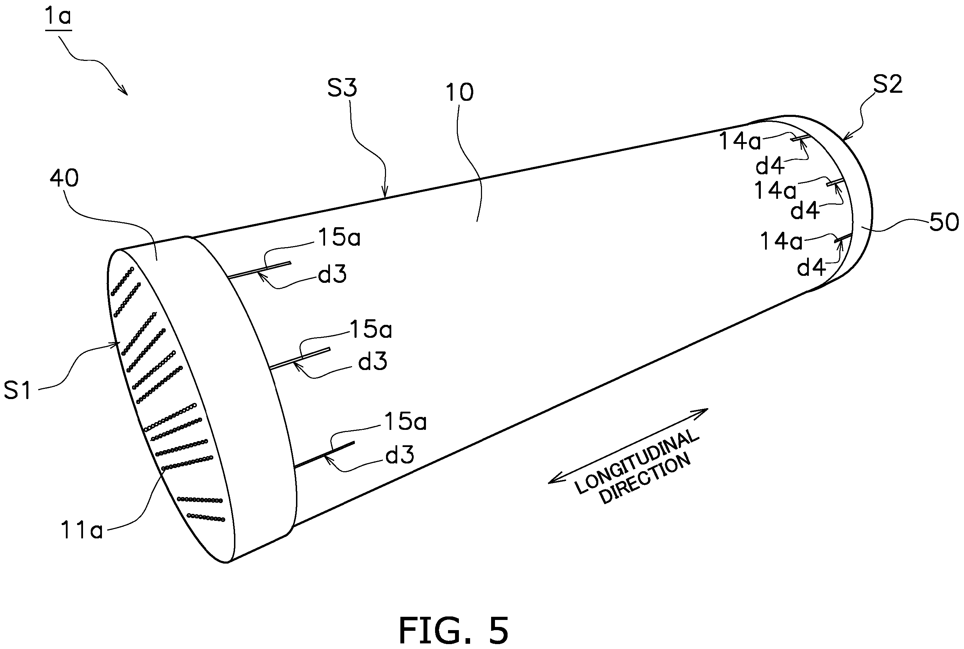

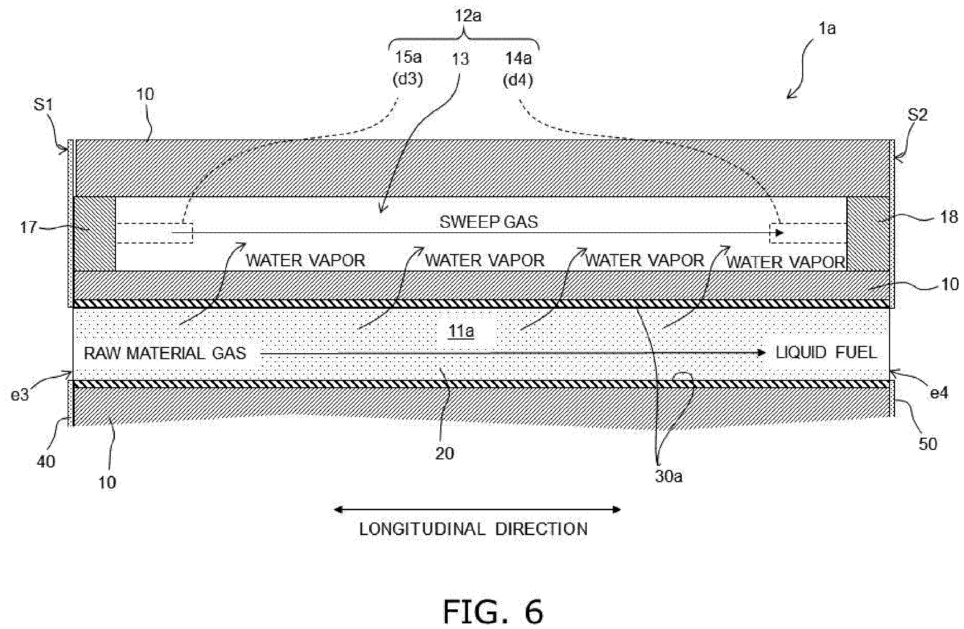

FIG. 5 is a perspective view of the reactor 1a. FIG. 6 is a cross-sectional view in the longitudinal direction of the reactor 1a.

As shown in FIG. 6, the porous support body 10 has third flow paths 11a and fourth flow paths 12a.

As shown in FIG. 6, the third flow paths 11a are formed along the longitudinal direction of the reactor 1a. The third flow paths 11a are through holes. The third flow paths 11a are open in the first end face S1 and the second end face S2 of the reactor 1a. The third flow paths 11a each have an inflow port e3 formed in the first end face S1 and an outflow port e4 formed in the second end face S2. The third flow paths 11a are provided on the non-permeation side of the separation membranes 30a. The raw material gas flows through the third flow paths 11a. The catalyst 20 is disposed in each third flow path 11a.

The fourth flow paths 12a are provided on the permeation side of the separation membranes 30a. The sweep gas for sweeping water vapor that has permeated through the separation membranes 30a flows through the fourth flow paths 12a.

Here, as shown in FIG. 6, the fourth flow paths 12a are each formed by the cell 13, an outflow slit 14a, and an inflow slit 15a.

As shown in FIG. 1, the outflow slits 14a are formed in one end portion in the longitudinal direction of the reactor 1a. The one end portion of the reactor 1a means, when the reactor 1a is equally divided into five in the longitudinal direction, a portion extending to two-fifths from one end on the side from which the liquid fuel flows out. The outflow slits 14a are formed along the short direction of the reactor 1a. The outflow slits 14a pass through the cells 13. Both ends of the outflow slits 14a are open in the side face S3. The outflow slits 14a each have a pair of exhaust ports d4 formed in the side face S3. The pair of exhaust ports d4 correspond to ends on one side of the fourth flow paths 12a in the longitudinal direction.

As shown in FIG. 1, the inflow slits 15a are formed in the other end portion of the reactor 1a in the longitudinal direction. The other end portion of the reactor 1a means, when the reactor 1a is equally divided into five in the longitudinal direction, a portion extending to two-fifths from the other end on the side to which the raw material gas flows in. The inflow slits 15a are formed along the short direction of the reactor 1a. The inflow slits 15a pass through the cells 13. Both ends of the inflow slits 15a are open in the side face S3. The inflow slits 15a each have a pair of inflow ports d3 formed in the side face S3. The pair of inflow ports d3 correspond to ends on the other side of the fourth flow paths 12a in the longitudinal direction.

As described above, in the reactor 1a, the positions of the inflow slits and the outflow slits are opposite to that of the reactor 1.

### Liquid fuel synthesis method using reactor 1a

The liquid fuel synthesis method using the reactor 1a will be described with reference to FIG. 6.

The liquid fuel synthesis method using the reactor 1a includes a step of causing the sweep gas to flow through the fourth flow paths 12a provided on the permeation side of the separation membranes 30a (one example of the second separation membrane) while causing the raw material gas to flow through the third flow paths 11a provided on the non-permeation side of the separation membranes 30a.

The raw material gas flows into the third flow paths 11a through the inflow ports e3 of the third flow paths 11a. In the third flow paths 1 1a, in accordance with the above formula (1), water vapor is generated together with the liquid fuel from the raw material gas. The synthesized liquid fuel flows out through the outflow ports e4 of the third flow paths 11a. The residual raw material gas that was not used in the conversion reaction may be mixed with the synthesized liquid fuel. The water vapor, which is one product, permeates through the separation membranes 30a and the porous support body 10, and then moves to the fourth flow paths 12a.

After flowing in through the inflow ports d3 of the inflow slits 15a, the sweep gas flows into the cells 13 through the inflow slits 15a. Next, the sweep gas that has flowed into the cells 13 takes in water vapor that has permeated through the separation membranes 30a, and flows through the cells 13 toward the outflow slits 14a, while absorbing the reaction heat generated by the conversion reaction. The sweep gas that has reached the outflow slits 14a is discharged from the exhaust ports d4 of the outflow slits 14a.

Here, as shown in FIG. 6, in the side view of the separation membranes 30a, the direction in which the sweep gas flows through the fourth flow paths 12a is same as the direction in which the raw material gas flows through the third flow paths 11a. That is, the sweep gas flowing through the fourth flow paths 12a flows in the direction parallel with the direction in which the raw material gas flows through the third flow paths 11a.

For this reason, the reaction heat can be efficiently absorbed from the upstream area of the third flow paths 11a (specifically, the area on the inflow ports d3 side with respect to the center in the longitudinal direction of the third flow paths 11a) where the reaction heat tends to be generated due to the high raw material gas concentration. As a result, the deterioration of the catalysts 20 and the separation membranes 30 can be suppressed, and thus the life of the reactor 1a can be extended.

### Reactor device 100

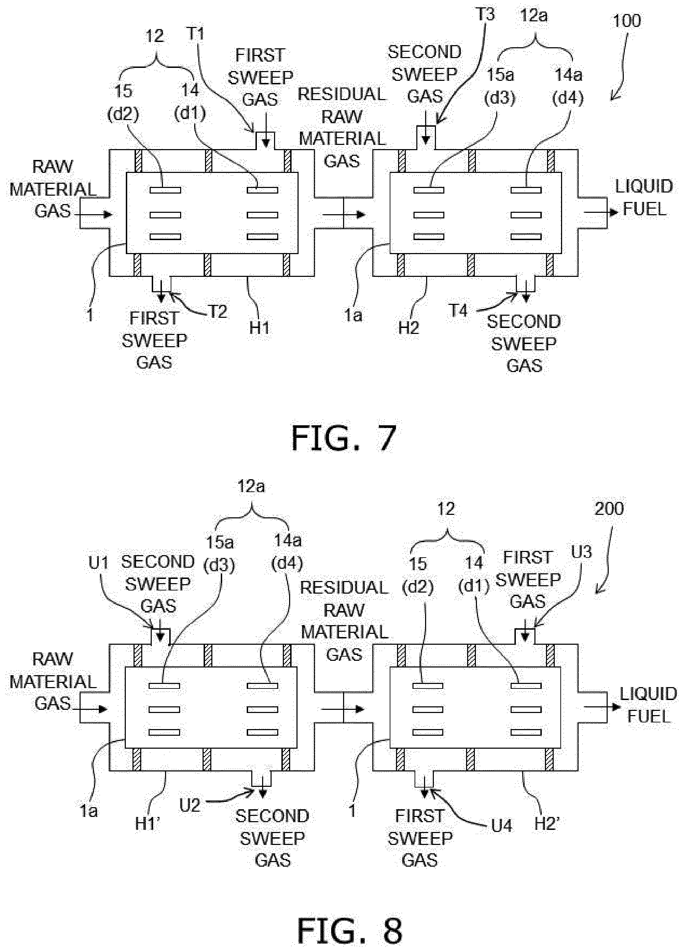

FIG. 7 is a schematic view of the reactor device 100. The reactor device 100 is one of the examples of usage of the reactor 1.

The reactor device 100 includes the reactor 1 (an example of a first reactor), the reactor 1a (an example of a second reactor), a first housing H1, and a second housing H2.

The reactor 1 is housed in the first housing H1. The raw material gas and a first sweep gas are supplied separately to the first housing H1. A space in the first housing H1 is partitioned such that the first sweep gas is not mixed with the raw material gas and the liquid fuel. The liquid fuel synthesized in the reactor 1 flows out from the first housing H1 together with the residual raw material gas.

The reactor 1a is disposed downstream (that is, in the rear) of the reactor 1. The reactor 1a is housed in the second housing H2. The liquid fuel containing the residual raw material gas and a second sweep gas are separately supplied to the second housing H2. A space in the second housing H2 is partitioned such that the second sweep gas is not mixed with the residual raw material gas and the liquid fuel. The liquid fuel synthesized in the reactor 1a flows out from the second housing H2.

Here, in the reactor 1, as shown in FIG. 4, in the side view of the separation membranes 30 (an example of a first separation membrane), the direction in which the first sweep gas flows through the second flow paths 12 is the same as the direction in which the raw material gas flows through the first flow paths 11. Accordingly, mixing of water vapor with the liquid fuel can be suppressed, and a liquid fuel of high quality can be obtained.

Also, in the reactor 1a, as shown in FIG. 6, in the side view of the separation membranes 30a (an example of a second separation membrane), the direction in which the second sweep gas flows through the fourth flow paths 12a is the same as the direction in which the raw material gas flows through the third flow paths 11a. Accordingly, the reaction heat can be efficiently absorbed, and the life of the reactor 1a can be extended.

In this manner, by combining the reactor 1 and the reactor 1a, both improvement in the quality of the liquid fuel and extension of the life of the reactor device can be achieved.

Note that, in FIG. 7, a sweep gas supply port T1 and a sweep gas exhaust port T2 that are formed in the first housing H1 are disposed on a straight line intersecting an axial center of the reactor 1 in the cross-sectional view. In this manner, since the lengths of the flow paths of the sweep gas that flows from the sweep gas supply port T1 to the sweep gas exhaust port T2 through the second flow paths 12 can be the same, deviation of the flow of the sweep gas can be suppressed. Note that the positional relationship between the sweep gas supply port T1 and the sweep gas exhaust port T2 can be changed as appropriate.

Similarly, in FIG. 7, a sweep gas supply port T3 and a sweep gas exhaust port T4 that are formed in the second housing H2 are disposed on a straight line intersecting an axial center of the reactor 1a in the cross-sectional view. In this manner, since the lengths of the flow paths of the sweep gas that flows from the sweep gas supply port T3 to the sweep gas exhaust port T4 through the fourth flow paths 12a can be the same, deviation of the flow of the sweep gas can be suppressed. Note that the positional relationship between the sweep gas supply port T3 and the sweep gas exhaust port T4 can be changed as appropriate.

### Reactor device 200

FIG. 8 is a schematic view of the reactor device 200. The reactor device 200 is one of the examples of usage of the reactor 1.

The reactor device 200 includes the reactor 1 (an example of a first reactor), the reactor 1a (an example of a second reactor), a first housing H1', and a second housing H2'.

The reactor 1a is disposed upstream (that is, in front) of the reactor 1. The reactor 1a is housed in the first housing H1'. The raw material gas and the second sweep gas are supplied separately to the first housing H1'. A space in the first housing H1' is partitioned such that the second sweep gas is not mixed with the raw material gas and the liquid fuel. The liquid fuel synthesized in the reactor 1 flows out from the reactor 1a together with the residual raw material gas.

The reactor 1 is housed in the second housing H2'. The liquid fuel containing the residual raw material gas and the first sweep gas are separately supplied to the second housing H2'. A space in the second housing H2' is partitioned such that the first sweep gas is not mixed with the residual raw material gas and the liquid fuel. The liquid fuel synthesized in the reactor 1 flows out from the second housing H2'.

Here, in the reactor 1a, as shown in FIG. 6, in the side view of the separation membranes 30a (an example of a second separation membrane), the direction in which the second sweep gas flows through the fourth flow paths 12a is the same as the direction in which the raw material gas flows through the third flow paths 11a. Accordingly, the reaction heat can be efficiently absorbed, and the life of the reactor device can be extended.

Here, in the reactor 1, as shown in FIG. 4, in the side view of the separation membranes 30 (an example of the first separation membrane), the direction in which the first sweep gas flows through the second flow paths 12 is the same as the direction in which the residual raw material gas flows through the first flow paths 11. Accordingly, mixing of water vapor with the liquid fuel can be suppressed, and a liquid fuel of high quality can be obtained.

In this manner, by combining the reactor 1 and the reactor 1a, both improvement in the quality of the liquid fuel and extension of the life of the reactor device can be achieved.

Note that, in FIG. 8, a sweep gas supply port U1 and a sweep gas exhaust port U2 formed in the first housing H1' are disposed on a straight line intersecting the axial center of the reactor 1a in the cross-sectional view. In this manner, since the lengths of the flow paths of the sweep gas that flows from the sweep gas supply port U1 to the sweep gas exhaust port U2 via the fourth flow paths 12a can be the same, deviation of the flow of the sweep gas can be suppressed. Note that, the positional relationship between the sweep gas supply port U1 and the sweep gas exhaust port U2 can be changed as appropriate.

Similarly, in FIG. 8, a sweep gas supply port U3 and a sweep gas exhaust port U4 formed in the second housing H2' are disposed on a straight line intersecting the axial center of the reactor 1 in the cross-sectional view. In this manner, since the lengths of the flow paths of the sweep gas that flows from the sweep gas supply port U3 to the sweep gas exhaust port U4 via the second flow paths 12 can be the same, deviation of the flow of the sweep gas can be suppressed. Note that, the positional relationship between the sweep gas supply port U3 and the sweep gas exhaust port U4 can be changed as appropriate.

### Modification of embodiment

Although an embodiment of the present invention has been described above, the present invention is not limited to the above embodiment, and various modifications can be made without departing from the gist of the invention.

### Modification 1

Although a monolith-type reactor 1 has been described as an example of a reactor according to the present invention, the mode of the reactor is not limited to this. The reactor according to the present invention may be a cylindrical reactor, for example.

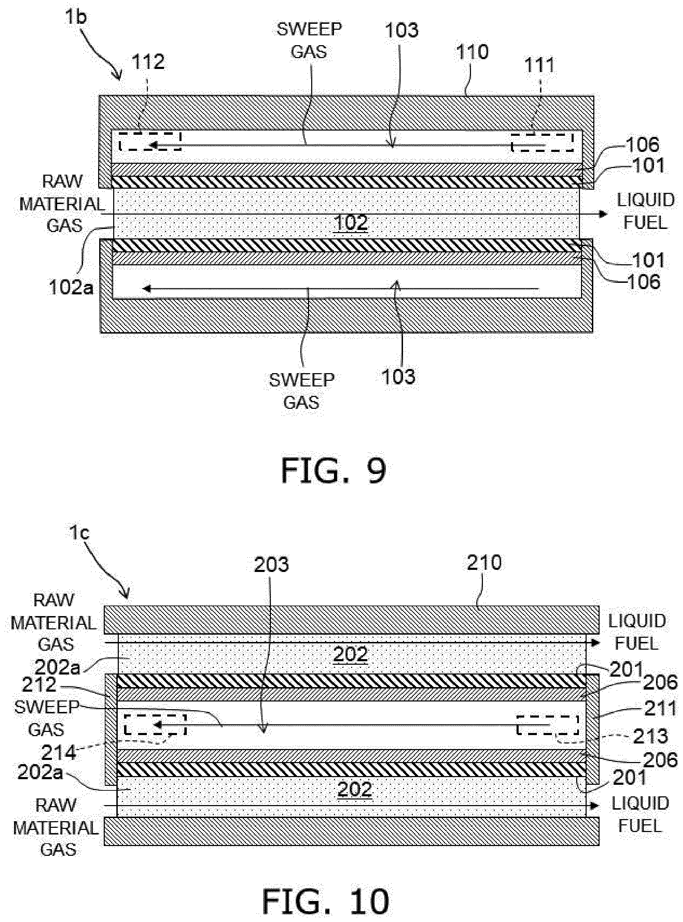

FIG. 9 is a cross-sectional view in the longitudinal direction of the cylindrical reactor 1b. The reactor 1b has a cylindrical separation membrane 101, a first flow path 102 provided on the inner side (non-permeation side) of the separation membrane 101 and through which the raw material gas flows, a second flow path 103 provided on the outer side (permeation side) of the separation membrane 101 and through which the sweep gas flows, and a catalyst 102a disposed in the first flow path 102.

The separation membrane 101 is supported by a cylindrical porous support body 106. The porous support body 106 is disposed on the inner side of a hollow columnar body 110, which is made of a highly-dense material. A space between the porous support body 106 and the hollow columnar body 110 is a second flow path 103. An inflow port 111 and an outflow port 112 for supplying and discharging the sweep gas are formed in the hollow columnar body 110.

In the side view of the separation membrane 101, the direction in which the sweep gas flows through the second flow path 103 is opposite to the direction in which the raw material gas flows through the first flow path 102. With this cylindrical reactor 1b as well, by causing the sweep gas to flow in the opposite direction to the raw material gas, mixing of water vapor with the liquid fuel can be suppressed, and a liquid fuel of high quality can be obtained.

Note that although the separation membrane 101 may be disposed on either the inner circumferential face or the outer circumferential face of the porous support body 106, if there is a difference in total pressure between the raw material gas and the sweep gas, the separation membrane 101 is preferably disposed on the inner circumferential face of the porous support body 106 as shown in FIG. 9. In this manner, generation of a crack on the separation membrane 101 can be suppressed.

FIG. 10 is a cross-sectional view along the longitudinal direction of the cylindrical reactor 1c. The reactor 1c includes a cylindrical separation membrane 201, a first flow path 202 provided on the outer side (non-permeation side) of the separation membrane 201 and through which the raw material gas flows, a second flow path 203 provided on the inner side (permeation side) of the separation membrane 101 and through which the sweep gas flows, and a catalyst 202a disposed in the first flow path 202.

The separation membrane 201 is supported by a cylindrical porous support body 206. The separation membrane 201 is surrounded by a cylindrical body 210 made of a highly-dense material. A space between the separation membrane 101 and the cylindrical body 210 is a first flow path 202. Both ends of the separation membrane 201 and the porous support body 206 are closed by a pair of compact seal members 211 and 212. A space between the pair of compact seal members 211 and 212 is a second flow path 203. The reactor 1c includes an inflow port 213 and an outflow port 214 for supplying and discharging the sweep gas. The inflow slit 213 and the outflow slit 214 are continuous with the inner part of the porous support body 206 and the outer part of the cylindrical body 210.

In the side view of the separation membrane 201, the direction in which the sweep gas flows through the second flow path 203 is opposite to the direction in which the raw material gas flows through the first flow path 202. With this cylindrical reactor 1c as well, by causing the sweep gas to flow in the opposite direction to the raw material gas, mixing of water vapor with the liquid fuel can be suppressed, and a liquid fuel of high quality can be obtained.

Note that although the separation membrane 201 may be disposed on either the inner circumferential face or the outer circumferential face of the porous support body 206, if there is a difference in total pressure between the raw material gas and the sweep gas, the separation membrane 201 is preferably disposed on the outer circumferential face of the porous support body 206 as shown in FIG. 10. In this manner, generation of a crack on the separation membrane 201 can be suppressed.

Note that a cylindrical reactor may be used as the reactor 1a.

### Modification 2

Although a monolith-type reactor 1 has been described as an example of the reactor according to the present invention in the above embodiment, the configuration of the reactor 1 can be changed as appropriate. For example, it has been described that the second flow paths 12 each include the pair of inflow ports d1 and the pair of exhaust ports d2, but the number and position of these can be changed as appropriate.

### Modification 3

Although the inflow slits 14 are formed in one end portion (a portion extending to two-fifths from one end on the side from which the liquid fuel flows out) of the reactor 1 in the above embodiment, at least part of the inflow slits 14 may be formed in one end portion of the reactor 1. Similarly, although the outflow slits 15 are formed in the other end portion (a portion extending to two-fifths from the other end on the side to which the liquid fuel flows in) of the reactor 1 in the above embodiment, at least part of the outflow slits 15 may be formed in the other end portion of the reactor 1. Note that at least part of one of or both the inflow slits 14 and the outflow slits 15 is preferably formed in a portion extending to one fifth from the end of the reactor 1. Since this makes it possible to enlarge the flowing range of the sweep gas, water vapor can be smoothly moved over a wider area from the downstream areas of the first flow paths 11 to the upstream area of the second flow paths 12.

### Modification 4

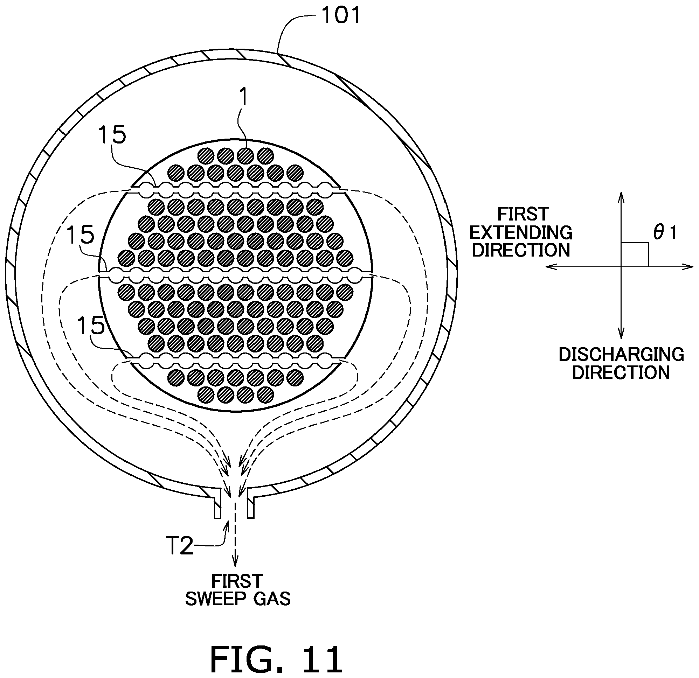

FIG. 11 is a cross-sectional view of a first housing H1 shown in FIG. 7. FIG. 11 shows a cross-sectional view orthogonal to the axial center of the reactor 1.

As shown in FIG. 11, a first extending direction in which the outflow slits 15 extend in the reactor 1 is preferably inclined or orthogonal with respect to a sweep gas discharging direction in which the sweep gas is discharged to the outside from the sweep gas exhaust port formed in the first housing H1. Specifically, an angle θ1 of the first extending direction with respect to the discharging direction is preferably 45° or more and 135° or less. In this manner, deviation of the flow of gas from opening portions on both sides of the outflow slits 15 to the sweep gas exhaust port can be suppressed, and thus deviation of the flow of the sweep gas can be suppressed.

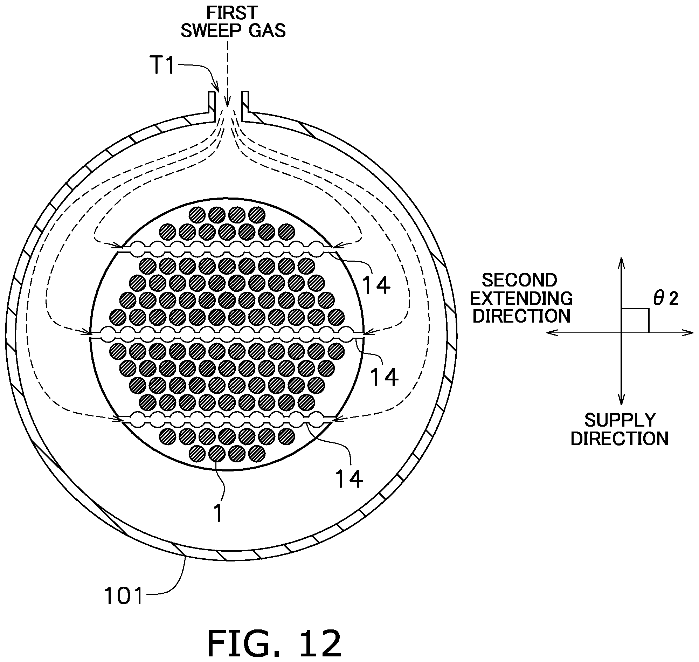

FIG. 12 is a cross-sectional view of the first housing H1 shown in FIG. 7. FIG. 11 shows a cross section orthogonal to the shaft center of the reactor 1.

As shown in FIG. 11, a second extending direction in which the inflow slits 14 extend in the reactor 1 is preferably inclined or orthogonal with respect to a sweep gas supply direction in which the sweep gas is supplied from the sweep gas supply port formed in the first housing H1. Specifically, an angle θ2 of the second extending direction with respect to the supply direction is preferably 45° or more and 135° or less. In this manner, deviation of the flow of gas from the sweep gas supply port to opening portions on both sides of the inflow slits 14 can be suppressed, and thus deviation of the flow of the sweep gas can be suppressed.

Note that the manners in which the sweep gas flows, which are shown in FIGS. 11 and 12, are preferably applied to the second housing H2 shown in FIG. 7, and the first housing H1' and the second housing H2' shown in FIG. 8.

### Modification 5

Although the separation membranes 30 are permeable to water vapor that is one of products of the conversion reaction of the raw material gas to the liquid fuel in the above embodiment, the present invention is not limited thereto. The separation membranes 30 may be permeable to the liquid fuel itself generated through the conversion reaction of the raw material gas to the liquid fuel. In this case as well, the reaction equilibrium of the above formula (1) can be shifted to the product side.

Also, in the case where the separation membranes 30 are permeable to the liquid fuel, even in the case the liquid fuel is generated through the reaction in which water vapor is not generated (for example, 2H₂ + CO ↔ CH₃OH), the reaction equilibrium can be shifted to the product side. Similarly, although the above has described that the separation membrane 30a is permeable to water vapor that is one of products of conversion reaction of the raw material gas to the liquid fuel, a configuration is also possible in which the separation membrane 30a may be permeable to the liquid fuel itself generated by the conversion reaction.

### REFERENCE SIGNS LIST

1 Reactor
10, 106, 206 Porous support body
11, 102, 202 First flow path
e1, 111, 213 Inflow port
e2, 112, 214 Outflow port
12 Second flow path
13 Cell
14 Inflow slit
d1 Inflow port
15 Outflow slit
d2 Exhaust port
20, 102a, 202a Catalyst
30, 101, 201 Separation membrane
40 First seal portion
50 Second seal portion

## Claims

1. A reactor comprising:
a separation membrane permeable to a product of conversion reaction of a raw material gas containing at least hydrogen and carbon dioxide to a liquid fuel;
a first flow path provided on a non-permeation side of the separation membrane, and configured to allow the raw material gas to flow through the first flow path;
a second flow path provided on a permeation side of the separation membrane, and configured to allow a sweep gas for sweeping water vapor permeating through the separation membrane to flow through the second flow path; and
a catalyst disposed in the first flow path and configured to promote the conversion reaction, wherein
in a side view of the separation membrane, a direction in which the sweep gas flows through the second flow path is opposite to a direction in which the raw material gas flows through the first flow path.

2. A reactor device comprising:
a first reactor; and
a second reactor disposed downstream of the first reactor,
the first reactor including:
a first separation membrane permeable to a product of conversion reaction of a raw material gas containing at least hydrogen and carbon dioxide to a liquid fuel;
a first flow path provided on a non-permeation side of the first separation membrane, and configured to allow the raw material gas to flow through the first flow path;
a second flow path provided on a permeation side of the first separation membrane, and configured to allow a first sweep gas for sweeping water vapor permeating through the first separation membrane to flow through the second flow path; and
a first catalyst disposed in the first flow path and configured to promote the conversion reaction, and
the second reactor including:
a second separation membrane permeable to a product of conversion reaction of a residual raw material gas to the liquid fuel, the residual raw material gas flowing out from the first reactor;
a third flow path provided on a non-permeation side of the second separation membrane, and configured to allow the residual raw material gas to flow through the third flow path;
a fourth flow path provided on a permeation side of the second separation membrane, and configured to allow a second sweep gas for sweeping water vapor permeating through the second separation membrane to flow through the fourth flow path; and
a second catalyst disposed in the third flow path and configured to promote the conversion reaction, and
in a side view of the first separation membrane, a direction in which the first sweep gas flows through the second flow path is opposite to a direction in which the raw material gas flows through the first flow path, and
in a side view of the second separation membrane, a direction in which the second sweep gas flows through the fourth flow path is the same as a direction in which the residual raw material gas flows through the third flow path.

3. A reactor device comprising:
a first reactor;
a second reactor disposed upstream of the fist reactor,
the first reactor including:
a first separation membrane permeable to a product of conversion reaction of a residual raw material gas to a liquid fuel, the residual raw material gas flowing out from the second reactor;
a first flow path provided on a non-permeation side of the first separation membrane, and configured to allow the residual raw material gas to flow through the first flow path; and
a second flow path provided on a permeation side of the first separation membrane, and configured to allow a first sweep gas for sweeping water vapor permeating through the first separation membrane to flow through the second flow path, and
the second reactor includes:
a second separation membrane permeable to a product of conversion reaction of a raw material gas containing at least hydrogen and carbon dioxide to a liquid fuel;
a third flow path provided on a non-permeation side of the second separation membrane, and configured to allow the raw material gas to flow through the third flow path; and
a fourth flow path provided on a permeation side of the second separation membrane, and configured to allow the second sweep gas for sweeping water vapor permeating through the second separation membrane to flow through the fourth flow path, and
in a side view of the first separation membrane, a direction in which the first sweep gas flows through the second flow path is opposite to a direction in which the residual raw material gas flows through the first flow path, and
in a side view of the second separation membrane, a direction in which the second sweep gas flows through the fourth flow path is the same as a direction in which the raw material gas flows through the third flow path.

4. A liquid fuel synthesis method using a reactor including a separation membrane permeable to a product of conversion reaction of a raw material gas containing at least hydrogen and carbon dioxide to a liquid fuel, the method comprising:
a step of causing a sweep gas to flow through a second flow path provided on a permeation side of the separation membrane, while causing the raw material gas to flow through a first flow path provided on a non-permeation side of the separation membrane, wherein
a catalyst configured to promote the conversion reaction is disposed in the first flow path, and
in a side view of the separation membrane, a direction in which the sweep gas flows through the second flow path is opposite to a direction in which the raw material gas flows through the first flow path.

5. A liquid fuel synthesis method using:
a first reactor including a first separation membrane permeable to a product of conversion reaction of a raw material gas containing at least hydrogen and carbon dioxide to a liquid fuel; and
a second reactor including a second separation membrane permeable to a product of conversion reaction of a residual raw material gas to a liquid fuel, the residual raw material gas flowing out from the first reactor,
the method including:
a step of causing a first sweep gas to flow through a second flow path provided on a permeation side of the first separation membrane, while causing the raw material gas to flow through a first flow path provided on a non-permeation side of the first separation membrane, and
a step of causing a second sweep gas to flow through a fourth flow path provided on a permeation side of the second separation membrane, while causing the residual raw material gas to flow through a third flow path provided on a non-permeation side of the second separation membrane, wherein
catalysts configured to promote the conversion reaction are disposed in the first flow path and the third flow path,
in a side view of the first separation membrane, a direction in which the first sweep gas flows through the second flow path is opposite to a direction in which the raw material gas flows through the first flow path, and
in a side view of the second separation membrane, a direction in which the second sweep gas flows through the fourth flow path is the same as a direction in which the residual raw material gas flows through the third flow path.

6. A liquid fuel synthesis method using:
a second reactor including a second separation membrane permeable to a product of conversion reaction of a raw material gas containing at least hydrogen and carbon dioxide to a liquid fuel; and
a first reactor including a first separation membrane permeable to a product of conversion reaction of a residual raw material gas to a liquid fuel, the residual raw material gas flowing out from the second reactor,
the method including:
a step of causing a second sweep gas to flow through a fourth flow path provided on a permeation side of the second separation membrane, while causing the raw material gas to flow through a third flow path provided on a non-permeation side of the second separation membrane, and
a step of causing a first sweep gas to flow through a second flow path provided on a permeation side of the first separation membrane, while causing the residual raw material gas to flow through a first flow path provided on the non-permeation side of the first separation membrane, wherein
catalysts configured to promote the conversion reaction are disposed in the first flow path and the third flow path,
in a side view of the first separation membrane, a direction in which the first sweep gas flows through the second flow path is opposite to a direction in which the raw material gas flows through the first flow path, and
in a side view of the second separation membrane, a direction in which the second sweep gas flows through the fourth flow path is the same as a direction in which the residual raw material gas flows through the third flow path.
